# EUROPEAN PATENT APPLICATION

(11) **EP 4 796 553 A1**
(43) Date of publication of application: **26.08.2026**
(21) Application number: 25159008.9
(22) Date of filing: 20.02.2025
(51) Int. Cl.: C07F 17/00, C07C 13/465, B01J 31/22

(54) **AN IMPROVED PROCESS FOR THE MANUFACTURE OF RACEMIC ANSA-METALLOCENES**

(71) Applicant: LANXESS Organometallics GmbH, 59192 Bergkamen (DE)
(72) Inventor: HOLTRICHTER-RÖßMANN, Thorsten, 59227 Ahlen (DE); LIEDTKE, Claus Günter, 59368 Werne (DE); MAUS, Silvia, 59379 Selm (DE)
(74) Representative: Deblon, Jörg-Stephan

(57) **Abstract**

The present invention relates to an improved process for the separation of racemic mixtures of bridged, stereorigid metallocenes of group 4 elements from specific solvent mixtures.

## Description

The present invention relates to an improved process for the separation of racemic mixtures of bridged, stereorigid metallocenes of group 4 elements from specific solvent mixtures.

Metallocenes based on optionally substituted cyclopentadienes, indenes and fluorenes, in particular cationic group 4 metallocene derivatives are known to efficiently catalyze olefin polymerization in combination with co-catalysts such as aluminoxanes and modified aluminoxanes.

Where chiral stereorigid metallocene racemates are employed in such polymerizations (see EP 320,762 A) stereoregular poly-α-olefins can be obtained in high yields while the respective meso forms of the metallocenes have shown not to be suitable for this task.

As a consequence there were made quite some attempts to synthesize the racemic forms in pure form and high yields.

US 5,017,714 discloses the preparation of silicon bridged metallocenes as oils from which the racemic and meso forms can be separated by various solvent extraction and/or crystallization steps.

EP 426,643 A discloses the preparation of metallocenes, which are stated to be of sufficient purity for subsequent use as a catalyst for the polymerization of olefins without further purification, by a solid-solid reaction of a transition metal salt and a powder of the solid reaction product of the ligand with an alkyllithium in a non-polar hydrocarbon liquid. Solid mixtures of lithium salt and the metallocene are recovered.

In US 5,556,997 the reaction of di-lithiated bis-indenyl-dimethylsilane with complexes of zirconium tetrachloride and chelating diamines is disclosed whereby high yields of racemic metallocenes and only small amounts of the meso form could be obtained.

A similar approach is reported in WO99/12943 where adducts of e.g. group 4 element halides with oligo- or poly-ethers or the respective thioethers are reacted with deprotonated metallocene ligands.

US 6,365,769 discloses a process for the preparation of dimethylsilyl-bis(indenyl)zirconium dichloride with a ratio of racemic/meso of 32:1 by di-lithiation of bis-indenyl-dimethylsilane with 20 wt.-% butyllithium in toluene, subsequent reaction with zirconium tetrachloride followed by intense workup and washing. For the analogous hafnium compound a racemic/meso ratio of 36:1 was reported.

In an alternative approach disclosed in DE 4406109 the preparation of pure racemic dimethylsilyl-bis(indenyl)zirconium dichloride and the analogous hafnium compound is performed by reaction of bis-2-indenyl-dimethylsilane with tetrabutyltin chloride, complete removal of the solvent and subsequent reaction with zirconium tetrachloride and hafnium tetrachloride respectively. The mechanistic aspects and an explanation for the high stereospecific synthesis are highlighted in M. Hüttenhofer et al, Angew. Chem, 1998, Vol. 100, No. 16, p. 2378 - 2380.

However a disadvantage of all processes listed above and known so far is that multistep work-up processes are required to only obtain the desired racemic compounds. Larger amounts of the respective meso compounds are typically of no commercial value and have to be disposed off.

Therefore, there was still a need and to develop a process which allows, while avoiding the disadvantages described, to predominantly prepare the racemic forms of chiral bridged stereorigid metallocenes in high yields.

A process has now been found for the preparation of compounds of formula (I) 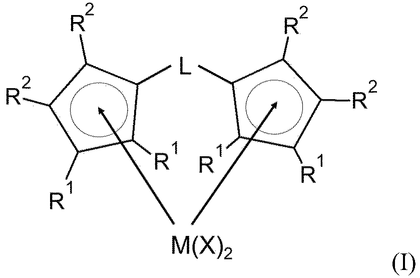 in their racemic form
wherein
L denotes a linker selected from the group consisting of -Si(R³)₂-, -C(R³)₂-, -C(R³)₂C(R³)₂-and BR³-
   wherein
R³ in case of -C(R³)₂- and-C(R³)₂C(R³)₂-denotes hydrogen, alkyl or phenyl and
   in case of -Si(R³)₂- and BR³ denotes alkyl or phenyl or
   two residues R³, where present, jointly denote alkane-di-yl,
whereby preferably R³
   in case of -C(R³)₂- denotes hydrogen, methyl or phenyl and
   in case of-C(R³)₂C(R³)₂- denotes hydrogen
   in case of-Si(R³)₂- and BR³ denotes methyl or ethyl
R¹ and R² independently of each other denote hydrogen, alkyl, phenyl or naphthyl which is optionally further substituted once, twice or more than twice by alkyl or phenyl, or two adjacent residues R¹ or two adjacent residues R² together and independently of the residues of the second cyclopentadienyl-moiety denote a benzene or thiophene ring fused to the cyclopentadienyl-moiety, whereby the fused benzene or thiophene ring is either further substituted by one, two, three or four phenyl or alkyl substituents or not
   under the proviso that R¹ and R² are selected such that they allow formation of racemic and meso forms of compounds of formula (I), which just for illustration excludes for example that all R¹ and R² are identical
M denotes a Lanthanoide, Chromium, Molybdenum, Tungsten, Titanium, Zirconium or Hafnium, preferably Lanthanum, Cerium, Neodymium, Chromium, Molybdenum, Tungsten, Zirconium or Hafnium, more preferably Zirconium, or Chromium or Molybdenum or Tungsten, more preferably Zirconium or Hafnium,
X denotes a halide such as fluoride, chloride, bromide or iodide, preferably fluoride, chloride or bromide and more preferably fluoride and chloride or an alkoxide of formula O-R wherein R denotes C₁-C₈-alkyl which may be once or twice substituted by phenyl which is optionally further substituted once, twice or more than twice by an C₁-C₄-alkyl or phenyl, preferably R denotes methyl or ethyl
the process comprising the step of
A) contacting a mixture comprising compounds of formula (I) in their racemic form and the meso form and an organic solvent comprising at least one ether at least one aiding compound selected from the group consisting of acylchlorides, esters and nitriles.

The scope of the invention encompasses all combinations of substituent definitions, parameters and illustrations set forth above and below, either in general or within areas of preference or preferred embodiments, with one another, i.e., also any combinations between the particular areas and areas of preference.

Whenever used herein the terms "including", "e.g.", "such as" and "like" are meant in the sense of "including but without being limited to" or "for example without limitation", respectively.

As used herein, and unless specifically stated otherwise **alkyl** may be straight-chained, cyclic either in part or as a whole, branched or unbranched.

Preferably, alkyl is C₁-C₈-alkyl. The term C₁-C₈-alkyl indicates that the straight-chained, cyclic either in part or as a whole, branched or unbranched alkyl substituent contains from 1 to 8 carbon atoms excluding the carbon atoms of optionally present substituents to the C₁-C₈-alkyl substituent. Specific examples of C₁-C₈-alkyl include methyl, ethyl, n-propyl, isopropyl, n-butyl, tert.-butyl, n-pentyl, cyclopentyl, cyclohexyl, n-hexyl, n-heptyl, n-octyl, iso-octyl, whereby methyl, ethyl, isopropyl, n-butyl, tert.-butyl and cyclopentyl are preferred.

The term phenyl which is optionally further substituted once, twice or more than twice by alkyl or phenyl preferably denotes phenyl, o-, m-, p-tolyl, 2,6-dimethylphenyl, 2,4-dimethylphenyl, 2,4,6-trimethylphenyl and p-phenyl-phenyl.

According to the invention a mixture comprising compounds of formula (I) in their racemic form and the meso form and an organic solvent comprising at least one ether are contacted is contacted with at least one compound selected from the group consisting of acylchlorides, esters and nitriles.

In one embodiment the term mixtures typically denote solutions or suspensions, preferably suspensions.

In another embodiment an organic solvent comprising at least one ether denotes
- ethers or mixtures of ethers, whereby the ethers are preferably selected from the group consisting of diethylether, tert.-butyl-methyl ether, tetrahydrofuran, methyl-tetrahydrofuran, dioxane, dimethoxyethane, diethoxymethane and tert.-amyl methyl ether and are even more preferably selected from the group consisting of diethylether, tert.-butyl-methylether tetrahydrofuran, dioxane dimethoxyethane, diethoxymethane and yet even more preferably tetrahydrofurane.
- mixtures of at least one ether and at least one aliphatic or aromatic hydrocarbon whereby the ether or the ethers are preferably selected from those described above including their preferences and the at least one aliphatic or aromatic hydrocarbon is preferably selected from the group consisting of benzene toluene, o-,p-,m-xylene and pentanes, hexanes and heptanes including but not limited to their linear branched and cyclic isomers either alone or in combination. In a preferred embodiment the mol ratio of the ether(s) employed and the amount of aliphatic or aromatic solvent forming part of the organic solvent is at least 100, preferably 100 to 500, more preferably from 100 to 400 and even more preferably from 150 to 400 and

The aiding compounds are selected from the group consisting of acylchlorides, esters and nitriles.

In one embodiment acylchlorides denote the following acylchlorides: (C₁-C₈-alkyl)-(C=O)Cl, preferably acetylchloride.

In one embodiment esters denote the following esters: (C₁-C₈-alkyl)-(C=O)-O(C₁-C₈-alkyl) preferably ethylacetate.

In one embodiment nitrile denote the following nitriles: (C₁-C₈-alkyl)-CN, benzonitrile and benzylnitrile, preferably acetonitrile.

Preferred compounds of formula (I) include
rac-1,2-ethylenebis(indenyl)-zirconium(IV) dichloride,
rac-1,2-ethylenebis(indenyl)-hafnium(IV) dichloride,
rac-1,2-ethylenebis(tetrahydroindenyl)-zirconium(IV) dichloride (CAS 100 163 29-9),
rac-1,2-ethylenebis(tetrahydroindenyl)-hafnium(IV) dichloriderac-dimethylsilyl-bis-(1-indenyl)-zirconium(IV)-dichloride,
rac-di rac-dimethylsilyl-bis-(1-tetrahydroindenyl)-zirconium (IV)-dichloride (CAS 126642-97-5),
rac-di rac-dimethylsilyl-bis-(1-tetrahydroindenyl)-hafnium (IV)-dichloride,
rac-dimethylsilyl-bis(2-methylindenyl)zirconium dibromide,
rac-dimethylsilyl-bis(2-methyl-4-phenylindenyl)zirconium dichloride,
rac-dimethylsilyl-bis[2-methyl-4-(1-naphthyl)indenyl]zirconium difluoride,
rac-dimethylsilyl-bis[2-methyl-4-(1-naphthyl)indenyl]hafnium dichloride,
rac-dimethylsilyl-bis(2-methyl-4,5-benzoindenyl)zirconium dichloride,
rac-dimethylsilyl-bis(4,5-benzoindenyl)zirconium dichloride,
rac-dimethylsilyl-bis(2-methyl-4,6-diisopropylindenyl)zirconium difluoride,
rac-dimethylsilyl-bis(2-ethylindenyl)zirconium dichloride,
rac-dimethylsilyl-bis(2ethyl-4-phenylindenyl)zirconium dichloride,
rac-dimethylsilyl-bis(2-ethyl-4-phenylindenyl)hafnium dichloride,
rac-dimethylsilyl-bis[2-ethyl-4-(1-naphthyl)indenyl]zirconium dichloride,
rac-dimethylsilyl-bis(2-ethyl-4,5-benzoindenyl)zirconium dichloride,
rac-dimethylsilyl-bis(4,5-benzoindenyl)zirconium dichloride,
rac-dimethylsilyl-bis(2-ethyl-4,6-diisopropylindenyl)zirconium dichloride,
rac-dimethylsilyl-bis(2-ethyl-4,6-dimethylindenyl)zirconium dichloride,
rac-dimethylsilyl-bis(2,4,6-trimethylindenyl)zirconium dichloride,
rac-dimethylsilyl-bis(2,4,6-trimethylindenyl)hafnium dichloride,
(1H-inden-2-yl)dimethyl-silyl-(2,3,4,5-tetramethylcyclopenta-2,4-dienyl)zirconium dichloride, dimethyl-silyl-(2-phenyl-1H-inden-1-yl)(2,3,4,5,-tetramethylcyclopenta-2,4-dienyl) zirconium dichloride, dimethyl-silyl-(2-methyl-4-phenyl-1-H-inden-1-yl)(2,3,4,5-tetramethylcyclopenta-2,4-dienyl) zirconium dichloride, dimethyl-silyl-(3-phenyl-1-H-inden-1-yl)(2,3,4,5-tetramethylcyclopenta-2,4-dienyl) zirconium dichloride, (2-isopropyl-1H-inden-1-yl)dimethyl-silyl-(2,3,4,5-tetramethylcyclopenta-2,4-dienyl) zirconium dichloride, (1H-inden-2-yl)dimethyl(2,3,4,5-tetramethyl-cyclopenta-2,4-dienyl) zirconium dichloride, dimethyl-silyl-(1-methyl-2-phenyl-1-H-inden-3-yl)(2,3,4,5-tetramethyl-cyclopenta-2,4-dienyl)zirconium dichloride, dimethyl-silyl-(1-methyl-2-phenyl-1 H-inden-3-yl)(2,3,4,5-tetramethyl-cyclopenta-2,4-dienyl)hafnium dichloride, (1H-inden-2-yl)dimethyl-silyl-(2,3,4,5-tetramethyl-cyclopenta-2,4-dienyl)hafnium dichloride, dimethyl-silyl-(2-phenyl-1H-inden-1-yl)(2,3,4,5-tetramethylcyclopenta-2,4-dienyl) hafnium dichloride, dimethyl-silyl-(2-methyl-4-phenyl-1-H-inden-1-yl)(2,3,4,5-tetramethylcyclopenta-2,4-dienyl) hafnium dichloride, Dimethyl-silyl-(3-phenyl-1-H-inden-1-yl)(2,3,4,5-tetramethylcyclopenta-2,4-dienyl) hafnium dichloride, (2-isopropyl-1H-inden-1-yl)dimethyl-silyl-(2,3,4,5-tetramethyl-cyclopenta-2,4-dienyl) hafnium dichloride and dimethylsilyl-(2,5-dimethyl-7H-cyclopenta[1,2-b;4,3-b']dithiophen-7-yl)-(2,4,7-trimethyl-1H-inden-1-yl)-zirconium dichloride, wherebydimethylsilyl-bis-(1-indenyl)-zirconium(IV)-dichloride, rac-1,2-ethylenebis(indenyl)-zirconium(IV) dichloride, rac-1,2-ethylenebis(tetrahydroindenyl)-zirconium(IV) dichloride (CAS 100 163 29-9), rac-dimethylsilyl-bis-(1-indenyl)-zirconium(IV)-dichloride, and rac-di rac-dimethylsilyl-bis-(1-tetrahydroindenyl)-zirconium (IV)-dichloride (CAS 126642-97-5) are even more preferred.

Within the meaning of the invention contacting may occur with or without active mixing, whereby active mixing is preferred and includes for example stirring or microsound assisted mixing or any other techniques known to those skilled in the art.

The contacting may be for a duration of 1 minute to 168 hours, preferably 30 minutes to 48 hours. Longer times typically do not add any advantage.

The temperature during the contacting may be for example from -20°C up to the boiling point of the lowest boiling component of the reaction mixture resulting from the contacting under the selected contacting pressure, preferably from 15°C to 70°C.

The pressure during contacting is for example from 500 hPa to 5 MPa, preferably ambient pressure.

In a further step B), the desired racemic compounds of formula (I) are isolated from the reaction mixture resulting from the contacting of the mixtures comprising compounds of formula (I) in their racemic form and the meso form and an organic solvent comprising at least one ether and at least one aiding compound according to the invention.

Such isolation may be effected by
i) optionally partially removing the organic solvent and / or the at least one aiding compound
ii) separation of the solids being present in the reaction mixture or formed upon partial removal of the solvent and / or the at least one aiding compound
by techniques known to those skilled in the art including sedimentation, centrifugation, filtration or decantation.

Without wanting to be bound by theory, it is assumed the aiding compounds are capable of isomerization of compounds of formula (I) by forming intermediate penta - or hexa-coordinated metal complexes and thereby shifting to equilibrium towards the typically less soluble racemic forms of compounds of formula (I).

In one embodiment the mixtures comprising compounds of formula (I) in their racemic form and the meso form and an organic solvent comprising at least one ether and employed in the present invention are prepared by adding the organic solvent to solid compounds of formula (I) being present as mixture of their racemic and meso form, for example comprising from 10 to 95 wt-%, preferably 50 to 90 wt.-% of the racemic form with regard to the total amount of compounds of formula (I).

In another embodiment the mixtures comprising compounds of formula (I) in their racemic form and the meso form and an organic solvent comprising at least one ether and employed in the present invention are prepared by
a) reacting compounds of formula (II) 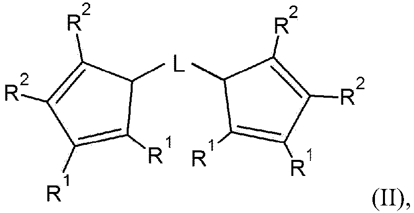 wherein L, R¹ and R² have the meaning defined above for formula (I)
   with 1.5 to 2.5 mol equivalents, preferably 1.8 to 2.2 mol equivalents, more preferably 1.9 to 2.1 mol equivalents and even more preferably 1.95 to 2.10 mol equivalents of at least one organolithium compound, whereby the organolithium compounds are employed in form of a solution in an aliphatic or aromatic solvent in the presence of at least one ether, preferably those selected from the group consisting of tetrahydrofuran, methyl-tetrahydrofuran, dioxane, dimethoxyethane, diethoxymethane, tert.-amyl methyl ether or mixtures thereof, preferably tetrahydrofuran, methyl-tetrahydrofuran or mixtures thereof and even more preferably tetrahydrofuran
   whereby the mol ratio of the ether(s) employed and compounds of formula (II) is from 5 to 70, preferably from 15 to 50 and more preferably from 20 to 35 and the mol ratio
   of the ether(s) employed and the overall amount of aliphatic or aromatic solvent forming part of the solution of the organolithium compound(s) is at least 100, preferably 100 to 500, more preferably from 100 to 400 and even more preferably from 150 to 400 and
b) subsequent reaction with compounds of formula (III)

   (D)₂M(X)₄

   wherein
   M and X have the meaning as defined for compounds of formula (I)
      and
   D is either missing or denotes a monoether, preferably tetrahydrofuran, a tertiary amine preferably trimethylamine or tetramethylethylenediamine ( TMEDA) or
   (D)₂ as a whole denotes a diether, preferably dioxane or dimethoxyethane or diethoxymethane.

In step a) compounds of formula (II) as defined above are employed.

Preferred compounds of formula (II) include 1,2-bisindenylethane, bis-(1-indenyl)-dimethyl-silane, (1H-inden-2-yl)dimethyl(2,3,4,5-tetramethylcyclopenta-2,4-dienyl)-silane, dimethyl(2-phenyl-1H-inden-1-yl)(2,3,4,5,-tetramethylcyclopenta-2,4-dienyl)silane,dimethyl(2-methyl-4-phenyl-1H-inden-1-yl)(2,3,4,5-tetramethylcyclopenta-2,4-dienyl)silane, dimethyl(3-phenyl-1-H-inden-1-yl)(2,3,4,5-tetramethylcyclopenta-2,4-dienyl)silane, (2-isopropyl-1H-inden-1-yl)dimethyl(2,3,4,5-tetramethylcyclopenta-2,4-dienyl)silane, (1H-inden-2-yl)dimethyl(2,3,4,5-tetramethylcyclopenta-2,4-dienyl)silane, dimethyl(1-methyl-2-phenyl-1-H-inden-3-yl)(2,3,4,5-tetramethylcyclopenta-2,4-dienyl)silane, dimethyl-bis(2-methylindenyl)silane, dimethyl-bis(2-methyl-4-phenylindenyl)silane, dimethyl-bis[2-methyl-4-(1-naphthyl)indenyl])silane, dimethyl-bis(2-methyl-4,5-benzoindenyl))silane, dimethyl-bis(2-methyl-4,6-diisopropylindenyl))silane, dimethyl-bis(2-ethylindenyl) )silane, dimethyl-bis(2-ethyl-4-phenylindenyl))silane, dimethyl-bis[2-ethyl-4-(1-naphthyl)indenyl])silane, dimethyl-bis(2-ethyl-4,5-benzoindenyl))silane, dimethyl-bis(4,5-benzoindenyl)silane, dimethyl-bis(2-ethyl-4,6-diisopropylindenyl))silane, dimethyl-bis(2-ethyl-4,6-dimethylindenyl)silane, dimethyl-bis(2,4,6-trimethylindenyl))silane, (2,5-dimethyl-7H-cyclopenta[1,2-b ;4,3-b']dithiophen-7-yl)-dimethyl-(2,4,7-trimethyl-1H-inden-1-yl)-silane,
whereby bis-(1-indenyl)-dimethyl-silane is even more preferred.

The compounds of formula (II) are reacted with solutions of organo-lithium compounds. Suitable organolithium compounds include phenyl-lithium, n-butyllithium, sec.-butyllithium, tert.-butyllithium and n-hexyllithium, whereby n-butyllithium is preferred. Solvents for such organolithium compounds include benzene, toluene and alkanes such as hexanes.

A preferred commercially available solution is a 10 M solution of n-butyl-lithium in hexanes.

The process according to the invention can be performed batchwise or continuously, preference being given to batchwise performance.

The reaction or residence times in step a) are for example from 5 minutes to 12 hours, preferably from 10 minutes to 4 hours.

The reaction or residence times in step b) are for example from 30 minutes to 24 hours, preferably from 30 minutes to 6 hours.

In one embodiment step a) is performed such that the compounds of formula (II) and the ether(s) are mixed to form a solution and to the resulting solution the solution of the organo-lithium compound is added. The addition may occur at once or in portions whereby addition in portions is preferred to maintain full control over the reaction temperature.

In step b) the compounds of formula (III) are added to the reaction mixture obtained according to step a). The addition may occur at once or in portions whereby addition at once is preferred.

The addition in step b) may occur at once or in portions whereby addition in portions is preferred to maintain full control over the reaction temperature.

The process according to steps a) and b) can be performed, for example, in any reactor allowing working under inert conditions known to those skilled in the art. Inert conditions shall in particular encompass working under a nitrogen and/or noble gas atmosphere and under exclusion of humidity. Suitable reactor types include stirred double wall glass reactors, stirred stainless steel, carbon steel and hastelloy reactors.

Step a) is performed, for example, at a reaction temperature of -80° to 30°C, preferably from -20°C to 30°C, more preferably from -10°C to 25°C.

Step b) is performed, for example, at a reaction temperature of -20° to 80°C, preferably from -10°C to 50°C, more preferably from 0°C to 25°C.

The reaction pressure is of no known significance and may, for example, be from 10 kPa to 10 MPa, whereby ambient pressure is preferred.

In step b) the compounds of formula (I) are formed.

Preferred compounds of formula (I) are those mentioned above.

The following examples are intended to illustrate the invention, but without limiting it thereto.

### Experimental part

### General

Acetylchloride, was obtained from VWR Germany and used as received.

ZrCl₄ and HfCl₄ employed herein were commercially obtained from VWR Germany and used as received. Tetrahydrofuran (THF) and toluene as employed herein were commercially obtained from Möller Chemie Germany and used as received.

The n-butyllithium employed herein was commercially obtained from Albemarle Germany and used as received. Bis-indenylsilane was obtained according to the procedure described in PCT Int. Appl. (1999), WO 9905152 A1 19990204, All manipulations were carried out using Schlenk based preparation techniques.

### A) The reactor

All experiments were performed in double-walled 2 L glass reactor with stainless steel paddle agitator connected to a protection gas flow and peripheral devices for the dosing of solids and liquid compounds. The reactor is connected to a Petite Fleur cryostate from Huber Germany.

### C) Product Analyses

The products were analyzed using a Bruker NMR-Spectrometer Avance II+ 400 using suitable pre-dried deuterated solvents for ¹H-NMR experiments, Zr and LiCl contents were measured using a Pectro Arcos/Blue or Agilent 7700 ICP-OES after a suitable acid base oxidative decomposition of the metallocene. Cl contents were measured by potentiometric titration using a Mettler Toledo T90 in combination with a Mettler DM141-SC.

### Preparation examples

### Comparative Example

86.6 g Bisindenylsilane (96 % purity, 288.2 mmol, 1 eq..) were dissolved in 649.5 g THF and cooled to -5°C. 41.7 g n-butyl lithium ( active content 90.8 mass-%, 590.8 mmol, 2,05 eq.) were added under the premise not to exceed 5 °C inside temperature. After the addition was finalized the mixture was heated to 20°C and stirred at 20°C for at least 30 minutes. Then the solution was cooled to 0°C.

In parallel 68.8 g ZrCl₄ (295.4 mmol, 1.03 eq.) were suspended in 172 g toluene and at 0 to 10°C 127.8 g THF (1772 mmol, 6.15 eq.) added. The mixture was stirred for 1 hour at 20°C and added at 0°C to the deprotonated bisindenylsilane in one portion. The solution was warmed to 20°C after the addition was completed and stirred for 10 hours at 20 to 25°C. The orange precipitate sedimented within 2 hours and 1000 ml of the supernatant solution were removed. 300 g of THF were added to the orange solid and the mixture stirred for one hour at 20°C. The product was isolated by filtration, washed 3 times with 100 g of THF each and dried in vacuo.
Yield: 49.5 g / 38 % / 100 % rac-isomer
¹H-NMR (400MHz, CDCl₃ ): δ (ppm) = 1.13 (s, 6H, Si(*CH*₃)), 6.09 (d, 2H, J₃ = 3.2 Hz, C*H*_{C5}), 6.92 (d, 2H, J₃ = 3.2 Hz, C*H*_{C5}), 7.07-7.11 (m, 2H, C*H*-_{Arom}), 7.35-7.37 (m, 2H, C*H*_{Arom}), 7.49 ( d, 2H, J₃ = 8.8 Hz, C*H*_{Arom}), 7.57 ( d, 2H, J₃ = 8.8 Hz, C*H*_{*-*Arom})
Elemental analysis: Cl content 15.9 mass-%, Zr content 20.4 mass-%, LiCl content 0.01 mass-%.

### Inventive Example 1

40.0 g Bisindenylethane (99.7 % purity, 154.4 mmol, 1 eq.) were dissolved in 300 g THF and cooled to -5°C. 22.3 g n-butyl lithium (active content 91.0 mass-%, 316.5 mmol, 2,05 eq.) were added under the premise not to exceed 25 °C inside temperature. After the addition was finalized the mixture was heated to 50°C and stirred at 50°C for at least 60 minutes. Then the solution was cooled to 0°C.

In parallel, 49.5 g ZrCl₄*2 THF Complex (131,2 mmol, 0.85 eq.) were suspended in 100 g THF. The mixture was stirred for 1 hour at 20°C and at 0°C the deprotonated bisindenylethane solution was added to the suspension in one portion. The solution was warmed to 20°C after the addition was completed, stirred for 1 hour at 20°C, heated to 50°C and stirred for further 4 hours at 50 °C. During cooling to 20°C 3.0 g acetyl chloride ( 38.6 mmol, 0.25 eq.) were added and the reaction stirred for at 8 hours at 20°C.

The yellow precipitate sedimented and the product was isolated by filtration, washed 3 times with 100 g of THF each and dried in vacuo.
Yield: 30.3 g / 55 % / 100 % rac-isomer
¹H-NMR (400MHz, CDCl₃ ): δ (ppm) = 3.73-3.81 (m, 4H, C*H₂-*C*H₂*))*,* 6.22 (d, 2H, J₃ = 3.2 Hz C*H*_{C5}), 6.61 (d, 2H, J₃ = 2.8 Hz, C*H*_{C5}), 7.20-7.36 (m, 4H, C*H*_{Arom}), 7.49 (d, 2H, J= 8.8 Hz, CH_{Arom}), 7.67 (d, 2H, J = 8.4 Hz, C*H*_{-Arom}),
Elemental analysis: Cl content 16.2 mass-%, Zr content 21.2 mass-%, LiCl content 0.5 mass-%.

### Preparation of ZrCl₄*2THF Complex

233.3 g ZrCl₄ were suspended in 485 mL dichloromethane and at 0 to 5°C 485 mL THF were slowly added. After the addition the mixture was stirred at 20 to 25°C for one hour and the dichloromethane then removed. To the almost dry remaining suspension 375 mL hexanes were added and the ZrCl₄*2THF complex isolated by filtration and dried via vacuum. The isolated product was used without further analysis.

## Claims

1. A process for the preparation of compounds of formula (I) 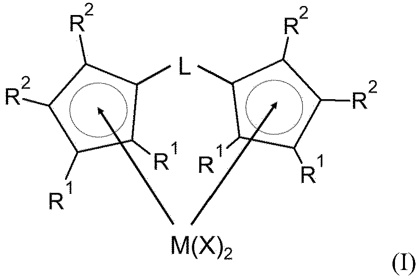 in their racemic form
wherein
L denotes a linker selected from the group consisting of -Si(R³)₂-, -C(R³)₂-, -C(R³)₂C(R³)₂-and BR³-
wherein
R³ in case of -C(R³)₂- and-C(R³)₂C(R³)₂-denotes hydrogen, alkyl or phenyl and
in case of -Si(R³)₂- and BR³ denotes alkyl or phenyl or
two residues R³, where present, jointly denote alkane-di-yl,
whereby preferably R³
in case of -C(R³)₂- denotes hydrogen, methyl or phenyl and in case of-C(R³)₂C(R³)₂- denotes hydrogen
in case of-Si(R³)₂- and BR³ denotes methyl or ethyl
R¹ and R² independently of each other denote hydrogen, alkyl, phenyl or naphthyl which is optionally further substituted once, twice or more than twice by alkyl or phenyl, or two adjacent residues R¹ or two adjacent residues R² together and independently of the residues of the second cyclopentadienyl-moiety denote a benzene or thiophene ring fused to the cyclopentadienyl-moiety, whereby the fused benzene or thiophene ring is either further substituted by one, two, three or four phenyl or alkyl substituents or not
under the proviso that R¹ and R² are selected such that they allow formation of racemic and meso forms of compounds of formula (I).
M denotes a Lanthanoide, Chromium, Molybdenum, Tungsten, Titanium, Zirconium or Hafnium, preferably Lanthanum, Cerium, Neodymium, Chromium, Molybdenum, Tungsten, Zirconium or Hafnium, more preferably Zirconium, or Chromium or Molybdenum or Tungsten, more preferably Zirconium or Hafnium,
X denotes a halide such as fluoride, chloride, bromide or iodide, preferably fluoride, chloride or bromide and more preferably fluoride and chloride or an alkoxide of formula O-R wherein R denotes C₁-C₈-alkyl which may be once or twice substituted by phenyl which is optionally further substituted once, twice or more than twice by an C₁-C₄-alkyl or phenyl, preferably R denotes methyl or ethyl
the process comprising the step of
A) contacting a mixture comprising compounds of formula (I) both, in their racemic form and the meso form, and an organic solvent comprising at least one ether at least one aiding compound selected from the group consisting of acylchlorides, esters and nitriles.

2. The process according to claim 1, wherein the aiding compounds are selected from the group consisting of the following acyl chlorides: (C₁-C₈-alkyl)-(C=O)Cl, preferably acetylchloride or the following esters: (C₁-C₈-alkyl)-(C=O)-O(C₁-C₈-alkyl), preferably ethylacetate or the following nitriles: (C₁-C₈-alkyl)-CN, benzonitrile and benzylnitrile, whereby acetonitrile is preferred.

3. The process according to claim 1 or 2, wherein the aiding compound is acetyl chloride.

4. The process according to anyone of claims 1 to 3, wherein the compounds of formula (I) are selected from the group consisting of
rac-1,2-ethylenebis(indenyl)-hafnium(IV) dichloride, rac-1,2-ethylenebis(tetrahydroindenyl)-zirconium(IV) dichloride (CAS 100 163 29-9),
rac-1,2-ethylenebis(tetrahydroindenyl)-hafnium(IV) dichloriderac-dimethylsilyl-bis-(1-indenyl)-zirconium(IV)-dichloride,
rac-di rac-dimethylsilyl-bis-(1-tetrahydroindenyl)-zirconium (IV)-dichloride (CAS 126642-97-5),
rac-di rac-dimethylsilyl-bis-(1-tetrahydroindenyl)-hafnium (IV)-dichloride,
rac-dimethylsilyl-bis(2-methylindenyl)zirconium dibromide,
rac-dimethylsilyl-bis(2-methyl-4-phenylindenyl)zirconium dichloride,
rac-dimethylsilyl-bis[2-methyl-4-(1-naphthyl)indenyl]zirconium difluoride,
rac-dimethylsilyl-bis[2-methyl-4-(1-naphthyl)indenyl]hafnium dichloride,
rac-dimethylsilyl-bis(2-methyl-4,5-benzoindenyl)zirconium dichloride,
rac-dimethylsilyl-bis(4,5-benzoindenyl)zirconium dichloride,
rac-dimethylsilyl-bis(2-methyl-4,6-diisopropylindenyl)zirconium difluoride,
rac-dimethylsilyl-bis(2-ethylindenyl)zirconium dichloride,
rac-dimethylsilyl-bis(2ethyl-4-phenylindenyl)zirconium dichloride,
rac-dimethylsilyl-bis(2-ethyl-4-phenylindenyl)hafnium dichloride,
rac-dimethylsilyl-bis[2-ethyl-4-(1-naphthyl)indenyl]zirconium dichloride,
rac-dimethylsilyl-bis(2-ethyl-4,5-benzoindenyl)zirconium dichloride,
rac-dimethylsilyl-bis(4,5-benzoindenyl)zirconium dichloride,
rac-dimethylsilyl-bis(2-ethyl-4,6-diisopropylindenyl)zirconium dichloride,
rac-dimethylsilyl-bis(2-ethyl-4,6-dimethylindenyl)zirconium dichloride,
rac-dimethylsilyl-bis(2,4,6-trimethylindenyl)zirconium dichloride,
rac-dimethylsilyl-bis(2,4,6-trimethylindenyl)hafnium dichloride,
(1H-inden-2-yl)dimethyl-silyl-(2,3,4,5-tetramethylcyclopenta-2,4-dienyl)zirconium dichloride, dimethyl-silyl-(2-phenyl-1H-inden-1-yl)(2,3,4,5,-tetramethylcyclopenta-2,4-dienyl) zirconium dichloride, dimethyl-silyl-(2-methyl-4-phenyl-1-H-inden-1-yl)(2,3,4,5-tetramethylcyclopenta-2,4-dienyl) zirconium dichloride, dimethyl-silyl -(3-phenyl-1-H-inden-1-yl)(2,3,4,5-tetramethylcyclopenta-2,4-dienyl) zirconium dichloride, (2-isopropyl-1H-inden-1-yl)dimethyl-silyl-(2,3,4,5-tetramethylcyclopenta-2,4-dienyl) zirconium dichloride, (1H-inden-2-yl)dimethyl(2,3,4,5-tetramethyl-cyclopenta-2,4-dienyl) zirconium dichloride, dimethyl-silyl-(1-methyl-2-phenyl-1-H-inden-3-yl)(2,3,4,5-tetramethyl-cyclopenta-2,4-dienyl)zirconium dichloride, dimethyl-silyl-(1-methyl-2-phenyl-1 H-inden-3-yl)(2,3,4,5-tetramethyl-cyclopenta-2,4-dienyl)hafnium dichloride, (1H-inden-2-yl)dimethyl-silyl-(2,3,4,5-tetramethyl-cyclopenta-2,4-dienyl)hafnium dichloride, dimethyl-silyl-(2-phenyl-1H-inden-1-yl)(2,3,4,5-tetramethylcyclopenta-2,4-dienyl) hafnium dichloride, dimethyl-silyl-(2-methyl-4-phenyl-1-H-inden-1-yl)(2,3,4,5-tetramethylcyclopenta-2,4-dienyl) hafnium dichloride, Dimethyl-silyl-(3-phenyl-1-H-inden-1-yl)(2,3,4,5-tetramethylcyclopenta-2,4-dienyl) hafnium dichloride, (2-isopropyl-1H-inden-1-yl)dimethyl-silyl-(2,3,4,5-tetramethyl-cyclopenta-2,4-dienyl) hafnium dichloride and dimethylsilyl-(2,5-dimethyl-7H-cyclopenta[1,2-b;4,3-b']dithiophen-7-yl)-(2,4,7-trimethyl-1H-inden-1-yl)-zirconium dichloride, wherebydimethylsilyl-bis-(1-indenyl)-zirconium(IV)-dichloride, rac-1,2-ethylenebis(indenyl)-zirconium(IV) dichloride, rac-1,2-ethylenebis(tetrahydroindenyl)-zirconium(IV) dichloride (CAS 100 163 29-9), rac-dimethylsilyl-bis-(1-indenyl)-zirconium(IV)-dichloride, and rac-di rac-dimethylsilyl-bis-(1-tetrahydroindenyl)-zirconium (IV)-dichloride (CAS 126642-97-5) are even more preferred.

5. The process according to anyone of claims 1 to 4, wherein the contacting occurs for a duration of 1 minute to 168 hours, preferably 30 minutes to 48 hours.

6. The process according to anyone of claims 1 to 5, wherein the contacting occurs at a temperature of -20°C up to the boiling point of the lowest boiling component of the reaction mixture resulting from the contacting under the selected contacting pressure, preferably from 15°C to 70°C.

7. The process according to anyone of claims 1 to 6, wherein the contacting occurs at a pressure of 500 hPa to 5 MPa, preferably at ambient pressure.

8. The process according to anyone of claims 1 to 7, wherein in a further step B), the desired racemic compounds of formula (I) are isolated from the reaction mixture resulting from the contacting of the mixtures comprising compounds of formula (I) in their racemic form and the meso form and an organic solvent comprising at least one ether and at least one aiding compound.

9. The process according to claims 8, wherein the isolation is effected by
i) optionally partially removing the organic solvent and / or the at least one aiding compound
ii) separation of the solids being present in the reaction mixture or formed upon partial removal of the solvent and / or the at least one aiding compound
by sedimentation, centrifugation, filtration or decantation.

10. The process according to anyone of claims 1 to 9, wherein the mixtures comprising compounds of formula (I) in their racemic form and the meso form and an organic solvent comprising at least one ether and employed in the proc ess according to anyone of claims 1 to 9 are prepared by adding the organic solvent to solid compounds of formula (I) being present as mixture of their racemic and meso form, for example comprising from 10 to 95 wt-%, preferably 50 to 90 wt.-% of the racemic form with regard to the total amount of compounds of formula (I).

11. The process according to anyone of claims 1 to 10, wherein the mixtures comprising compounds of formula (I) in their racemic form and the meso form and an organic solvent comprising at least one ether and employed in the present invention are prepared by
a) reacting compounds of formula (II) 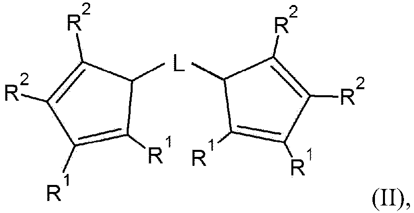 wherein L, R¹ and R² have the meaning defined above for formula (I) in claim 1
with 1.5 to 2.5 mol equivalents, preferably 1.8 to 2.2 mol equivalents, more preferably 1.9 to 2.1 mol equivalents and even more preferably 1.95 to 2.10 mol equivalents of at least one organolithium compound, whereby the organolithium compounds are employed in form of a solution in an aliphatic or aromatic solvent in the presence of at least one ether, preferably those selected from the group consisting of tetrahydrofuran, methyl-tetrahydrofuran, dioxane, dimethoxyethane, diethoxymethane, tert.-amyl methyl ether or mixtures thereof, preferably tetrahydrofuran, methyl-tetrahydrofuran or mixtures thereof and even more preferably tetrahydrofuran
whereby the mol ratio of the ether(s) employed and compounds of formula (II) is from- 5 to 70, preferably from 15 to 50 and more preferably from 20 to 35 and the mol ratio of the ether(s) employed and the overall amount of aliphatic or aromatic solvent forming part of the solution of the organolithium compound(s) is at least 100, preferably 100 to 500, more preferably from 100 to 400 and even more preferably from 150 to 400 and
b) subsequent reaction with compounds of formula (III)
(D)₂M(X)₄
wherein
M and X have the meaning as defined for compounds of formula (I)
and
D is either missing or denotes a monoether, preferably tetrahydrofuran, a tertiary amine preferably trimethylamine or tetramethylethylenediamine ( TMEDA) or
(D)₂ as a whole denotes a diether, preferably dioxane or dimethoxyethane or diethoxymethane.
